# EUROPEAN PATENT SPECIFICATION

(11) **EP 0 424 387 B1**
(45) Date of publication and mention of the grant of the patent: **14.09.1994**
(21) Application number: 89903533.1
(22) Date of filing: 14.07.1988
(51) Int. Cl.: A61M 1/00, A61M 37/00

(54) **INTRAMEDULLARY CATHETER**
MARKKATHETER
CATHETER INTRAMEDULLAIRE

(43) Date of publication of application: 02.05.1991
(73) Proprietor: BOARD OF REGENTS, THE UNIVERSITY OF TEXAS SYSTEM, Austin, Texas 78701 (US)
(72) Inventor: VON HOFF, Daniel, D., San Antonio, TX 78230 (US); KUHN, John, G., San Antonio, TX 78250 (US); LEIGHTON, Paul, W., Thiensdale, WI 53092 (US); WAKEMAN, Howard, M., Thiensdale, WI 53092 (US)
(74) Representative: Clifford, Frederick Alan
(86) International application number: US8802391
(87) International publication number: WO9000412

(56) References cited:
- EP-A- 0 103 081
- EP-A- 0 134 745
- WO-A-88/06023
- US-A- 2 426 535
- US-A- 3 750 667
- US-A- 4 494 535
- US-A- 4 772 261

## Description

### 1. Field of the Invention

This invention relates to a device for delivering fluids to the vascular system through the bone marrow cavity. The application moreover describes a method of implanting such a device, and a method of using such a device. In particular, the device comprises an intramedullary catheter which is implanted in a bone and covered by the skin, providing access for the administration of fluids through the bone marrow into the vascular system.

### 2. Description of the Relevant Art

Intravenous devices are commonly used for the delivery of fluids, such as drugs or the like, directly to the vascular system. Most hospital patients are fitted with an intravenous device to provide the physician with easy access to the vascular system for the administration of such fluids. The advantages of intravenous devices for quickly delivering medication to the vascular system are readily apparent, there are, however, a number of disadvantages.

In using intravenous devices for example, infection of the surrounding tissue (cellulitis) and systemic infection (bacteremia) can sometimes occur. Further, clotting of the vein (thrombosis) and accidental injection of the medication outside of the vein - causing extensive tissue destruction - also occur with some regularity. Perhaps the greatest problem with such intravenous devices is simply the procedure involved in inserting such devices. Often, locating the vein in which such an intravenous device can be placed is difficult, subjecting the patient to a painful ordeal as the doctor or nurse probes the area under the skin in an attempt to find a vein. All of these problems are particularly magnified in those patients requiring long-term (e.g. greater than two weeks) administration of medication.

As an answer to the well known deficiencies of conventional intravenous devices, several systems have been developed for use as an alternative. One such device is the Hichman Broviac silastic catheter which is tunneled under the skin, usually in the chest, and inserted into a large vein, usually the subclavian vein. Other such systems include the Port-a-cath (sold by Pharmacia-Deltec), Infus-a-port (sold by Infasaid), and the Mediport (sold by Norfolk) which are essentially cavity structures which are implanted under the skin and have a self-sealing septum. Medication is injected through a needle inserted through the skin and septum into the cavity. A catheter leads from the cavity to the vein to deliver the medication to the vein.

While such implant devices are sometimes a desirable alternative to an intravenous device, a number of problems still exist. For example, infection and clotting in the vein and catheter are still major problems. Further, these implant devices require minor surgery for insertion, and are nevertheless difficult to implant in children. Further, the bulges caused by such implant devices are cosmetically unappealing.

Perhaps the greatest difficulty with such implant devices is that because of the many complications that can arise, many such implant devices must be prematurely removed from the patient. Thus, while such implant devices are often a desirable alternative to intravenous devices, in practice many problems exist with such implant devices.

The invention consists in a device attachable to a bone for providing access for fluid delivery to the vascular system, the device comprising: an elongated, tubular conduit; attaching means disposed on the exterior surface of the conduit; and a head attached to one end of the conduit;
characterised in that the head includes a seal means comprising a silicone rubber self-sealing membrane overlying the conduit to permit insertion of a needle through the seal means to deliver fluid to the conduit for transport to the bone marrow and subsequent dispersion to the vascular system.

The device hereof provides convenient access to the vascular system without major infection, clotting, cosmetic, and insertion problems associated with intravenous devices and such implant devices. It is particularly appropriate where the patient requires long-term administration of medication.

U.S.Patent No. 3750667 (Psenichny) describes an elongate tubular device for intraosseous injection provided with longitudinal movable cover portions to prevent injection holes at an inner end becoming blocked with osseous tissue during placement, and further possessing a gland seal at the head of the outside tube. European Patent Application 103081 (Cullor) similarly shows an elongate tubular device for fitment into a body part to supply fluid thereto, but envisages that the fluid shall be supplied by means of a syringe fitting to a taper-fit adapter region of the tubular device. In neither case is there shown a design of implantable device with a self-sealing membrane at the outer end, as in the present invention.

Broadly speaking, the present invention contemplates a device, alternatively known as an intramedullary catheter or osteoport, which is in use attached to a bone for providing access for fluid delivery to the vascular system. The device includes a tubular conduit which is inserted through a bore in the bone in communication with the bone marrow cavity. A head is attached to one end of the conduit to lay adjacent the bone such that the surrounding skin can be closed about the head. The head includes a seal mechanism which overlies the conduit and permits the insertion of a needle through the seal mechanism to deliver the fluid or other medication to the conduit for transport to the bone marrow. It has been found that many medications delivered to the bone marrow are rapidly dispersed throughout the vascular system.

A method of fluid or drug delivery to the vascular system of a patient may be effected using such an intramedullary catheter. In the method of fluid delivery, the intramedullary catheter is implanted in a bone with the conduit in communication with the bone marrow cavity by drilling a bore into the bone and inserting the conduit into the bore. Preferably, the bore in the bone is tapped with threads and the conduit has threads along a portion such that the catheter is screwed (threadingly secured) into the bore. The drug or other fluid is injected through the overlying skin into the conduit for delivery to the bone marrow and transport to the vascular system.
FIGURE 1 represents a fragmentary anatomical sectional view of the iliac crest with an intramedullary catheter of the present invention implanted therein;
FIGURE 2 is a sectional view of an intramedullary catheter of the present invention;
FIGURE 3 is a top plan view of the head of the intramedullary catheter taken along line 3-3 of FIGURE 2; and
FIGURE 4 is an elevational view of the intramedullary catheter of the present invention with the sealing mechanism exploded for clarity.

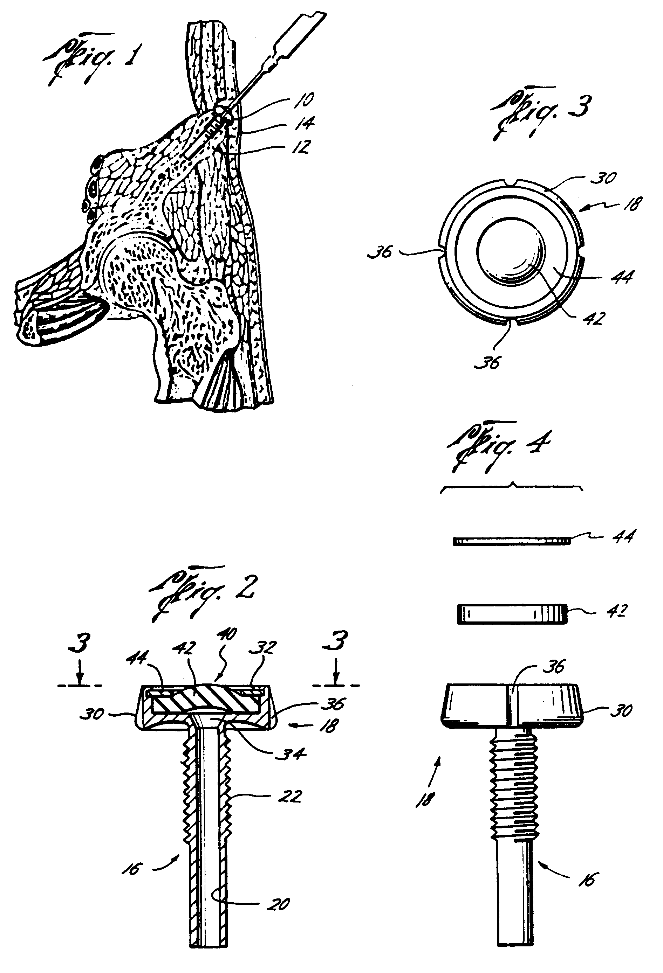

Turning now to the drawing, FIGURE 1 illustrates an intramedullary catheter 10 implanted in a bone 12 such as the anterior iliac crest, with the skin and surrounding tissue 14 overlying the catheter 10. As shown in FIGS. 2-4, the intramedullary catheter 10 broadly includes an elongated tubular conduit portion 16 and a head portion 18.

As can be seen readily from FIGURE 2, the conduit 16 is elongated and needle-like to define a passage 20. The outer portion of the conduit 16 includes a mechanism for attaching the conduit 16 to bone 12, which in the preferred embodiment comprises the threads 22. The distal end of the conduit 16 (remote from the head 18) is simply a flat surface, but in alternative embodiments may comprise a sharpened point.

The head 18 comprises an enlarged circular saucer 30 concentrically attached to the conduit 16 and preferably defining a cylindrical cavity 32. Structure defining a truncated cone-shaped bore 34 connects the cavity 32 with the passage 20. The outer periphery of the saucer 30 includes a plurality of circumferentially spaced apart notches 36 (see FIGS. 3 and 4).

A seal mechanism 40 is interfitted into the cavity 32 as shown in FIGURE 2. The seal mechanism includes a silicone rubber self-sealing membrane 42 which is complementarily dimensioned for sliding reception into the cavity 32. As can be appreciated from FIGURE 2, the membrane 42 is slightly enlarged relative to the cavity 32 so that the perimeter of the membrane 42 sealingly engages the internal walls of the saucer 30 defining the cavity 32, causing the central portion of the membrane 42 to bulge outwardly as shown in FIGURE 2. An annular retaining washer 44 is interfitted in an annular groove in the saucer walls defining the cavity 32. The washer 44 overlies the membrane 42, retaining the membrane 42 in position in the cavity 32.

The catheter 10 can be implanted in many different bones in the skeletal structure with the iliac crest 12 shown in FIGURE 1 a preferred entry location. In the method of implanting the catheter 10, a small incision in the skin 14 is made in the region of the iliac crest 12 and the iliac crest 12 is positively identified through the incision. After identification of the iliac crest, a bore is drilled through the bone 12 into communication with the marrow cavity as shown in FIGURE 1. An internal thread is tapped into the iliac crest 12 utilizing a thread size and tap used routinely in orthopedic practice for inserting pins in bones.

The catheter is inserted through the incision in the skin 14, with the distal end of the conduit 16 disposed in the threaded bore in the iliac crest 12. The catheter 10 is then screwed into the iliac crest 12 so that the catheter 10 is secured in the bone 12 by the threads 22. In the preferred method of implantation, a driving tool (not shown) is fitted to the catheter 10 and includes four spaced apart lugs which interfit into the notches 36, so that rotation of the driving tool screws the catheter 10 into the bone 12.

As can be seen in FIGURE 1, with the catheter 10 fully inserted in the bone 12, the head 18 abuts the external surface of the bone 12 and the skin 14 is closed over the head 18.

The use of the catheter 10 contemplates a fluid delivery method to the vascular system through the bone marrow cavity. As used in the present application, the term "fluids" is used broadly to include drugs and other medications. To deliver the fluids to the catheter 10, the region of the skin 14 surrounding the catheter 10 is prepared and the needle on the end of a conventional intravenous tubing is inserted through the skin 14 and into the membrane 42. Preferably, the distal tip of the needle is inserted through the membrane 14 and positioned somewhere in the region of the passage 20 proximate the bore 34. Typically, the needle includes a threaded or luer-type hub that can be connected to a variety of external delivery systems. Normally, the needle is connected by tubing to an infusion bottle.

In use, the drugs or other fluids are administered through the catheter 10 into the bone marrow cavity. It has been found that where drugs are injected into bone marrow cavity, the drugs are rapidly absorbed from the marrow cavity. In many instances, the drugs are delivered as rapidly to the vascular system through the marrow cavity as in direct infusion into a vein.

In practice, it is believed that the catheter 10 and methods of implantation and use offer many advantages over conventional drug delivery procedures. For example, there are no clotting problems in that the catheter 10 is not located in a vein, and similarly, the probability of infection is diminished. Once the catheter 10 is inserted, there are no cosmetic disfigurations and little or no chance for occlusion or migration of the catheter 10. The cost of inserting the catheter 10 is minimal, and it can be easily inserted in historically more difficult patients such as infants and geriatrics or other long term care patients. Finally, there is no chance for pneumothorax and no chance for leakage of toxic drugs outside of the desired location, as is possible with intravenous devices.

In summary, it is believed that the catheter 10 and the method of implantation and drug delivery which it permits offer a notable advance over the art. In particular, the catheter methods of the present invention represent a significant advance for patients requiring long term administration of fluids.

## Claims

1. A device (10) attachable to a bone for providing access for fluid delivery to the vascular system, the device comprising: an elongated, tubular conduit (16); attaching means disposed on the exterior surface of the conduit (16); and a head (18) attached to one end of the conduit (16);
characterised in that the head includes a seal means comprising a silicone rubber self-sealing membrane (42) overlying the conduit to permit insertion of a needle through the seal means to deliver fluid to the conduit (16) for transport to the bone marrow and subsequent dispersion to the vascular system.

2. The device (10) according to claim 1, wherein the attaching means comprises a plurality of threads (22) alone a portion of the exterior surface of the conduit (16).

3. The device (10) according to claim 1, wherein the head (18) comprises an enlarged circular saucer (30) defining a cavity (32) therein.

4. The device (10) according to claim 3, wherein the self-sealing membrane (42) is disposed within the cavity of the head (18).

5. The device (10) according to claim 4, wherein the head (18) includes a washer (44) adjoining the membrane (42) for retaining the membrane in the cavity (32).

6. The device (10) according to claims 2, wherein the saucer (30) is concentrically oriented relative to the conduit (16) and oriented generally perpendicular to the longitudinal axis of the conduit.

7. The device (10) according to claim 1, wherein the head includes a plurality of spaced apart notches (36) adapted for engaging a tool for screwing the device.

## Patentansprüche

1. Vorrichtung (10), die an einem Knochen befestigt werden kann, zur Ermöglichung der Einleitung eines Fluids in das Gefäßsystem, wobei die Vorrichtung folgende Elemente aufweist: eine längliche, rohrförmige Leitung (16); ein Befestigungsmittel, das auf der Außenfläche der Leitung (16) angeordnet ist, und ein Kopfteil (18), das an einem Ende der Leitung (16) befestigt ist;
dadurch gekennzeichnet, daß das Kopfteil ein Dichtungsmittel einschließt, das eine selbstdichtende Membran (42) aus Silikongummi aufweist, welche die Leitung überlagert, um die Einführung einer Nadel zur Einleitung eines Fluids in die Leitung (16) für den Transport zum Knochenmark und für die nachfolgende Dispersion auf das Gefäßsystem durch das Dichtungsmittel hindurch zu ermöglichen.

2. Vorrichtung (10) nach Anspruch 1, bei welcher das Befestigungsmittel eine Vielzahl von Gewindegängen (22) längs eines Abschnitts der Außenfläche der Leitung (16) aufweist.

3. Vorrichtung (10) nach Anspruch 1, bei welcher das Kopfteil (18) eine vergrößerte, runde Schale (30) aufweist, in der ein Hohlraum (32) gebildet wird.

4. Vorrichtung (10) nach Anspruch 3, bei welcher die selbstdichtende Membran (42) innerhalb des Hohlraums des Kopfteils (18) angeordnet ist.

5. Vorrichtung (10) nach Anspruch 4, bei welcher das Kopfteil (18) eine Dichtungsscheibe (44) einschließt, die an die Membran (42) angrenzt, um die Membran im Hohlraum (32) zu halten.

6. Vorrichtung (10) nach Anspruch 2, bei welcher die Schale (30) im Verhältnis zur Leitung (16) konzentrisch ausgerichtet ist und allgemein senkrecht zur Längsachse der Leitung ausgerichtet ist.

7. Vorrichtung (10) nach Anspruch 1, bei welcher das Kopfteil eine Vielzahl von räumlich getrennten Kerben (36) einschließt, die mit einem Werkzeug zusammenwirken können, um die Vorrichtung einzuschrauben.

## Revendications

1. Un dispositif (10) pouvant être fixé à un os pour établir un accès en vue d'amener un fluide au système vasculaire, le dispositif comprenant: un conduit tubulaire allongé (16); un moyen de fixation agencé sur la surface externe du conduit (16); et une tête (18) fixée à une extrémité du conduit (16);
caractérisé en ce que la tête englobe un moyen d'étanchéité comprenant une membrane auto-étanchéifiante en caoutchouc silicone (42) superposée au conduit, permettant l'insertion d'une aiguille à travers le moyen d'étanchéité, pour amener du fluide au conduit (16) en vue du transport de ce fluide vers la moelle osseuse et de sa dispersion ultérieure dans le système vasculaire.

2. Le dispositif (10) selon la revendication 1, dans lequel le moyen de fixation comprend plusieurs filets (22) le long d'une partie de la surface externe du conduit (16).

3. Le dispositif (10) selon la revendication 1, dans lequel la tête (18) comprend un godet circulaire agrandi (30) qui y définit une cavité (32).

4. Le dispositif (10) selon la revendication 3, dans lequel la membrane auto-étanchéifiante (42) est disposée dans la cavité de la tête (18).

5. Le dispositif (10) selon la revendication 4, dans lequel la tête (18) englobe une rondelle (44) adjacente à la membrane (42) pour retenir la membrane dans la cavité (32).

6. Le dispositif (10) selon la revendication 2, dans lequel le godet (30) est orienté concentriquement par rapport au conduit (16) et orienté de façon généralement perpendiculaire par rapport à l'axe longitudinal du conduit.

7. Le dispositif (10) selon la revendication 1, dans lequel la tête englobe plusieurs entailles espacées (36) pouvant s'engager dans un outil en vue du vissage du dispositif.
